Europäisches Patentamt

(19) European Patent Office

Office européen des brevets

(11) Publication number: **0 233 764**
**A1**

(12) # EUROPEAN PATENT APPLICATION

(21) Application number: 87301266.0

(22) Date of filing: 13.02.87

(51) Int. Cl.⁴: **C 12 N 15/00**
C 12 N 7/00, A 61 K 39/21

(30) Priority: 14.02.86 US 829517

(43) Date of publication of application:
26.08.87 Bulletin 87/35

(84) Designated Contracting States:
AT BE CH DE FR GB IT LI LU NL SE

(71) Applicant: THE UNITED STATES OF AMERICA
represented by The Secretary The United States
Department of Commerce
5285 Port Royal Road
Springfield Virginia 22161 (US)

(72) Inventor: Fisher, Amanda Gay
5514 Johnson Avenue
Bethesda, Maryland 20817 (US)

Gallo, Robert Charles M. D.
8513 Thornden Terrace
Bethesda, Maryland 20834 (US)

Wong-Staal, Flossie
3 Lynn Manor Court
Rockville, Maryland 20850 (US)

Josephs, Steven Francis
1903 Valley Stream Drive
Rockville, Maryland 20851 (US)

Feinberg, Mark B.
1651 Woodlawn Avenue
Palo Alto California 94303 (US)

(74) Representative: Jump, Timothy John Simon et al
F.J. Cleveland and Company 40-43 Chancery Lane
London WC2A 1JQ (GB)

(54) Plasmids which inhibit Human T-Cell Lymphotropic Virus Type III replication.

(57) Biologically competent clones pHXB2ΔSal-Sst and pHXB2ΔSal-RI are derivatives of Human T-Cell Lymphotropic Virus Type III molecular clones, and contain TAT-III gene defective genomes. These clones specifically inhibit the replication of HTLV-III virus, and are thus suitable for use in the study and treatment of Acquired Immune Deficiency Syndrome.

FIG.1

EP 0 233 764 A1

**Description**

## PLASMIDS WHICH INHIBIT HUMAN T-CELL LYMPHOTROPIC VIRUS TYPE III REPLICATION

The phenomena of transcriptional activation of viral genes by viral regulatory elements acting in cis and trans configurations has been described for DNA tumor viruses (for example, Khoury et al, E. J. Virol., Vol. 23, pp. 167-176 (1977)). Transactivator genes have also been discovered in retroviruses in man -- especially implicated in the expression of viral genes.

Human T-Cell Lymphotropic Virus Type III (HTLV-III) and related viruses are the presumed causal agents of Acquired Immune Deficiency Syndrome (AIDS) and associated conditions. The transactivator gene of HTLV-III (TAT-III), as characterized by functional mapping studies, is comprised of three exons. For additional description of HTLV-III and the transactivator gene, see Arya et al, Science, Vol. 229, pp. 69-73 (1985) and Sodroski et al, Science, Vol. 229, pp. 74-77 (1985).

The first exon of TAT-III is non-coding and extends 287 base pairs from the 5′ LTR. The second exon, located between the sor (short open reading) frame and envelope genes, encodes 72 amino acids and is crucial to transactivation. The third exon encodes only 14 amino acids and is located within the envelope gene, but in a different reading frame.

Extensive studies of the genomic structure of HTLV-III and related viruses have identified at least five open reading frames within the genome: gag, pol, sor, env, and 3′orf. The present application discloses the significance of a newly discovered sixth open reading frame, TAT-III, in HTLV-III replication. Part of the significance of the present invention is that the product of this newly identified gene is an absolute requirement for virus expression. Furthermore, deletion of TAT-III coding sequences in plasmids of the present invention results in plasmids which fail to produce, or express unusually low, amounts of virus. These plasmids, therefore, may be used in the development of an effective therapeutic approach to the treatment of AIDS and related disorders.

The present invention comprises plasmid clone constructs containing modifications in the TAT-III gene. These constructs are derived from a plasmid clone which contains full length HTLV-III provirus and produces infectious HTLV-III virions and cytopathic effects when transfected into normal T-lymphocytes. Clone pHXB2gpt of the present invention is modified to remove the major coding region of TAT-III to produce pHXB2ΔSal-Sst or to remove the splice acceptor sequences of TAT-III to produce pHXB2ΔSal-RI.

### DESCRIPTION OF THE FIGURES

Figure 1 illustrates the construction of plasmids pHXB2ΔSal-Sst and pHXB2ΔSal-RI.

Figure 2 shows complementation of TAT activity in H9 cells transfected with the HTLV-III plasmids of the present invention.

Construction of HTLV-III plasmids pHXB2ΔSal-Sst and pHXB2ΔSal-RI. An 11.5 Kb XbaI to HpaI fragment containing full length Human T-Cell Lymphotropic Virus Type III ( HTLV-III) proviral DNA is excised by known methods from an HTLV-III phage clone designated λ HXB2. This phage clone is a well known and widely used HTLV-III phage clone described in Shaw et al, Science, Vol. 226, pp. 1165-1171 (1984) and contains full-length integrated proviral DNA of HTLV-III. While phage clone λ HXB2 is the preferred clone of the present invention, other clones, such as λ HXB3, may be used without destroying the integrity of the present invention. Therefore, clones which contain the XbaI to HpaI fragment are included within the scope of this invention.

The XbaI to HpaI fragment obtained from λ HXB2 is then inserted by known methods into the BamHI and EcoRI sites of plasmid pSP65gpt. pSP65gpt is a well known plasmid vector, and is the preferred plasmid vector of the present invention. Other vectors may be used without changing the integrity of the claimed invention.

Insertion of the XbaI to HpaI fragment into plasmid vector pSP65gpt results in the formation of clone pHXB2gpt, containing the viral insert in the same transcriptional orientation as the E. coli XGPT gene. pHXB2gpt is then used for the construction of the plasmids of the present invention, pHXB2ΔSal-Sst and pHXB2ΔSal-RI. Deleting nucleotides 5367 through 5580 from pHXB2gpt results in construct pHXB2ΔSal-Sst, a plasmid lacking the entire first coding exon of TAT III (exon 2) including the initiating methionine. This construct is generated from pHXB2gpt by a SstI partial restriction digest followed by digestion to completion with SalI, end repair with T4 DNA polymerase, and reclosure with T4 DNA ligase.

Deleting nucleotides 5323 through 5367 from pHXB2gpt results in construct pHXB2ΔSal-RI, a plasmid lacking the splice acceptor sequence used in the generation of mature TAT-III messenger RNA. In this plasmid, the entire TAT-III coding sequence is unperturbed. This construct is generated from pHXB2gpt by an EcoRI partial restriction digest, followed by complete digestion with SalI, repair with the Klenow fragment of E. coli DNA polymerase, and reclosure with T4 ligase.

### EXAMPLES

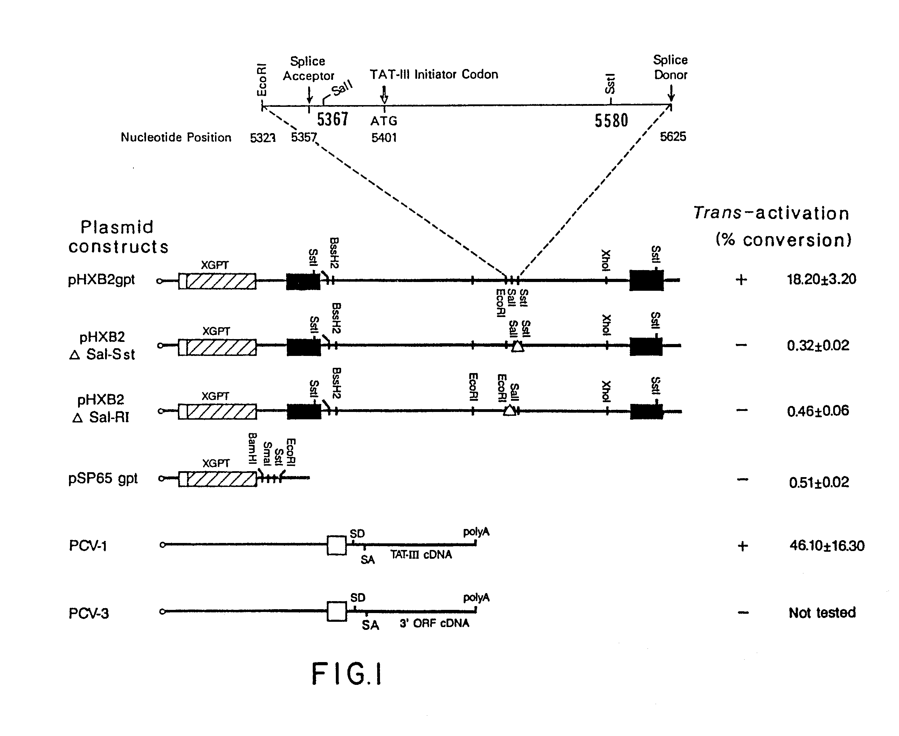

EXAMPLE 1. The extent to which the plasmids of the present invention (with deleted genomes) are capable of transactivating genes linked to the HTLV-III LTR's was assessed in a series of DEAE-mediated co-transfection experiments using pCD12CAT as an indicator. For a more detailed description of these types of experiments, see Queen et al, D. Cell., Vol. 33, pp. 741-748 (1983) . Chloramphenicol acetyltransferase (CAT) activity was determined 48 hours after transfection as the amount of cytoplasmic $^{14}$C-labelled chloramphenicol converted to acetylated metabolites, expressed as a percentage of the total. As shown in Figure 1, transfection of H9 cells with pHXB2gpt + pCD12CAT significantly augmented CAT activity (18.2%), as compared with transfection with pCD12CAT alone (0.03%) or pCD12CAT + pSP65gpt (0.5%), consistent with pHXBgpt containing a biologically functional TAT-III gene. Co-transfection of pCD12CAT with pHXB2ΔSal-Sst or pHXB2ΔSal-RI gave values of 0.32% and 0.46%, respectively.

EXAMPLE 2. To assess the capacity of TAT-III defective genomes for productive virus replication, constructs pHXB2ΔSal-Sst, pHXB2ΔSal-RI, pHXB2gpt, and pSP65gpt were introduced into H9 cells by protoplast fusion. This technique is used to transfect and stably express genes in eukaryotic cells. The experiments assessed the frequency of cells expressing HTLV-III p15 (gag related protein) following protoplast fusion. One week after transfection with pHXB2gpt, 1 to 15% of the H9 cells expressed HTLV-III p15; extracellular and budding virions were evident. The percentage of expressing cells increased to 80-90% within the first two weeks and was maintained at a high level throughout the duration of the experiment. In contrast, no virus or p15 expressing cells were detected after transfection with pHXB2ΔSal-Sst. These results indicate that the TAT-III gene is crucial for HTLV-III production. Southern blots prepared from these cells and probed for HTLV-III sequences showed the presence of linearized unintegrated plasmid DNA (a 17Kb band) and integrated HTLV-III sequences (a high relative molecular mass smear >23Kb). This shows that the failure of H9/pHXB2ΔSal-Sst cultures to produce virus was not due to failure to introduce this DNA into cells.

EXAMPLE 3. Transfection of clone pHXB2ΔSal-RI resulted in virus expression in two of three cases. In these, low level expression (<2% of cells) of p15 was demonstrated 2 weeks after transfection. Electron micrographs revealed small numbers of exclusively extracellular particles, many of which exhibitied unusual morphology as compared with typical virions. These aberrant particles may represent true defective particles or simply degenerating virons. Two to eight weeks after transfection the proportion of cells expressing p15 increased to around 30% and reached a plateau at 10-20% in successive weeks.

These results have two implications: first, the molecular clone pHXB2ΔSal-RI is capable of generating HTLV-III virus, but at markedly reduced levels as compared to the parental pHXB2gpt plasmid (cultures transfected with the former are estimated to contain 10 times fewer particles). It is therefore believed that the pHXB2ΔSal-RI clone, deprived of the normal splice acceptor used to generate TAT-III, is able to use alternative splice acceptor sites in the 5' region of the genome in order to generate mature TAT-III mRNA.

Second, the frequency of HTLV-III p15 positive cells in pHXB2ΔSal-RI transfected cultures did not increase to approach 100%. Using an in situ technique capable of detecting infrequent cells expressing as few as 20 to 60 HTLV-III RNA transcripts per cell, it was determined that 0% and 25% of cultures I and II (week 8) and 20% of culture III (week 4) expressed viral RNA. These results are comparable with immunofluorescence values and show that the virus generated from pHXB2ΔSal-RI is either defective or produced in too low amounts to efficiently spread through the culture.

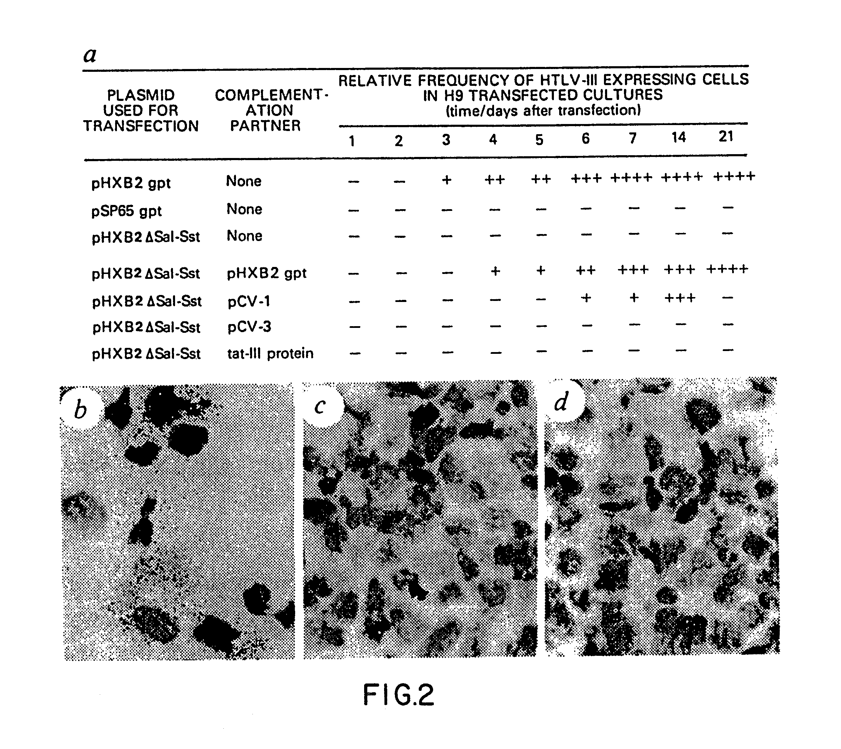

EXAMPLE 4. Complementation experiments were performed to determine whether the failure of TAT-defective clones to generate HTLV-III efficiently could be compensated by the provision of a functional TAT-III gene or the TAT-III protein itself (see Figure 2). H9 cells transfected with pHXB2gpt alone or in combination with pHXB2ΔSal-Sst expressed HTLV-III p15 and virus particles 3 to 4 days after transfection. This indicates that the simultaneous fusion of protoplasts containing different plasmids does not alter the efficiency of DNA transfection per se. HTLV-III expressing cells were not detected following transfection of pHXB2ΔSal-Sst alone or in combination with pCV-3 (a plasmid containing the complete cDNA of the 3'orf messenger RNA). However, following co-transfection of pHXB2ΔSal-Sst and pCV-1 (a plasmid containing the complete cDNA of the spliced TAT-III gene), HTLV-III p15 positive cells were apparent 6 (<0.01%) to 14 (2.8%) days after fusion and expressing cells confirmed in parallel in situ RNA studies. These results show that the capacity for virus expression can be transiently restored to a genome deprived of TAT-III by the provision of an unlinked, functional TAT-III gene.

## Claims

1. A molecular clone of Human T-Cell Lymphotropic Virus Type III, characterized in that it comprises a biologically competent clone of Human T-Cell Lymphotropic Virus Type III containing a TAT-III defective genome.

2. Recombinant clone pHXB2gpt, essentially characterized as containing an XbaI to HpaI viral DNA fragment obtained from Human T-Cell Lymphotropic Virus Type III molecular clone λ HXB2.

3. The recombinant clone of Claim 2, further characterized in that a major coding region corresponding to nucleotide sequences 5367-5580 is absent from said clone.

4. The recombinant clone of claim 2, further characterized in that splice acceptor sequences corresponding to nucleotide sequences 5323-5367 are absent from said clone.

5. A process for the production of a poorly infective but immunologically active Human T-Cell Lymphotropic Virus Type III, said process being characterized by removing a XbaI to HpaI fragment from Human T-Cell Lymphotropic Virus Type III phage clone pHXB2, inserting said fragment into BamHI and EcoRI sites of plasmid pSP65gpt to form clone pHXB2gpt, and deleting TAT III amino acid sequences from said clone pHXB2gpt.

6. The process of claim 5, further characterized in that the amino acid sequences deleted from said clone pHXB2gpt constitute exon 2 of the TAT III gene corresponding to nucleotide sequences 5367-5580.

7. The process of claim 5, further characterized in that the amino acid sequences removed from said clone pHXB2gpt constitute splice acceptor sequences corresponding to nucleotide sequences 5323-5367.

8. A therapeutic agent for inducing an immunological reaction to Human T-Cell Lymphotropic Virus Type III in human subjects, characterised by comprising a clone as claimed in any of claims 1, 2, 3 and 4.

9. A therapeutic agent for inducing an immunological reaction to Human T-Cell Lymphotropic Virus Type III in human subjects, characterised by comprising defective Human T-Cell Lymphotropic Virus Type III produced by a process as claimed in any of claims 5, 6 and 7.

10. The use of a clone as claimed in any of claims 1, 2, 3 and 4 in the manufacture of a medicament for the treatment or prevention of Acquired Immune Deficiency Syndrome (AIDS).

11. The use of a defective Human T-Cell Lymphotropic Virus Type III produced by a process as claimed in any of claims 5, 6 and 7 in the manufacture of a medicament for the treatment or prevention of Acquired Immune Deficiency Syndrome (AIDS).

FIG.I

*a*

| PLASMID USED FOR TRANSFECTION | COMPLEMENT-ATION PARTNER | RELATIVE FREQUENCY OF HTLV-III EXPRESSING CELLS IN H9 TRANSFECTED CULTURES (time/days after transfection) | | | | | | | | |
|---|---|---|---|---|---|---|---|---|---|---|
| | | 1 | 2 | 3 | 4 | 5 | 6 | 7 | 14 | 21 |
| pHXB2 gpt | None | − | − | + | ++ | ++ | +++ | ++++ | ++++ | ++++ |
| pSP65 gpt | None | − | − | − | − | − | − | − | − | − |
| pHXB2 ΔSal-Sst | None | − | − | − | − | − | − | − | − | − |
| pHXB2 ΔSal-Sst | pHXB2 gpt | − | − | − | + | + | ++ | +++ | +++ | ++++ |
| pHXB2 ΔSal-Sst | pCV-1 | − | − | − | − | − | + | + | +++ | − |
| pHXB2 ΔSal-Sst | pCV-3 | − | − | − | − | − | − | − | − | − |
| pHXB2 ΔSal-Sst | tat-III protein | − | − | − | − | − | − | − | − | − |

FIG.2

| | DOCUMENTS CONSIDERED TO BE RELEVANT | | EP 87301266.0 |

| Category | Citation of document with indication, where appropriate, of relevant passages | Relevant to claim | CLASSIFICATION OF THE APPLICATION (Int. Cl 4) |
|---|---|---|---|
| A | WO - A1 - 85/05 636 (DANA-FARBER CANCER INSTITUTE) <br><br> * Claims 1-5; page 21, last line - pages 23, line 5; fig. 5 * <br><br> -- | 1 | C 12 N 15/00 <br> C 12 N 7/00 <br> A 61 K 39/21 |
| D,A | SCIENCE, vol. 229, no. 4708, July 5, 1985 (Washington D.C.) <br><br> S.K.ARYA et al. "Trans-Activator Gene of Human T-Lymphotropic Virus Type III (HTLV-III)" pages 69-73 <br><br> * Totality * <br><br> -- | 1 | |
| D,A | SCIENCE, vol. 229, no. 4708, July 5, 1985 (Washington D.C.) <br><br> J.SODROSKI et al. "Location of the Trans-Activating Region of the Genome of Human T-Cell Lymphotropic Virus Type III" pages 74-77 <br> * Totality * <br> -- | 1 | TECHNICAL FIELDS SEARCHED (Int. Cl 4) <br><br> C 12 N <br> A 61 K |
| D,A | SCIENCE, vol. 226, no. 4679, December 7, 1984 (Washington D.C.) <br><br> G.M.SHAW et al. "Molecular Characterization of Human T-Cell Leukemia (Lymphotropic) Virus Type III in the Acquired Immune Deficiency Syndrome" pages 1165-1171 <br><br> * Fig. 2 * <br><br> ---- | 2 | |

The present search report has been drawn up for all claims

| Place of search | Date of completion of the search | Examiner |
|---|---|---|
| VIENNA | 07-05-1987 | WOLF |